# FASCICULE DE BREVET EUROPEEN

(11) **EP 3 170 461 B1**
(45) Date de publication et mention de la délivrance du brevet: **11.07.2018**
(21) Numéro de dépôt: 16198180.8
(22) Date de dépôt: 10.11.2016
(51) Int. Cl.: A61B 17/16

(54) **ANCILLAIRE DE RÉTROPERÇAGE ET ENSEMBLE DE PRÉPARATION OSSEUSE COMPRENANT UN TEL ANCILLAIRE DE RÉTROPERÇAGE**
HILFSMITTEL FÜR RÜCKSEITIGE BOHRUNG, UND GESAMTHEIT EINER KNOCHENVORBEREITUNG, DIE EIN SOLCHES HILFSMITTEL ZUR RÜCKSEITIGEN BOHRUNG UMFASST
BACK-DRILLING ANCILLARY AND BONE-PREPARATION ASSEMBLY COMPRISING SUCH A BACK-DRILLING ANCILLARY

(30) Priorité: 20.11.2015 FR 1561193
(43) Date de publication de la demande: 24.05.2017
(73) Titulaire: Amplitude, 26000 Valence (FR)
(72) Inventeur: POTEL, Jean-François, 31500 TOULOUSE (FR); VINCIGUERRA, Bruno, 64600 ANGLET (FR); VERDOT, François-Xavier, 42000 SAINT ETIENNE (FR); HEMERY, Xavier, 44600 SAINT-NAZAIRE (FR); FOURATI, ELIES, 51500 RILLY-LA-MONTAGNE (FR); BADET, Roger, 69003 LYON (FR); MABIT, Christian, 87000 LIMOGES (FR); COSTE, Cédric, 87110 LE VIGEN (FR); BADATCHEFF, François, 49100 ANGERS (FR)
(74) Mandataire: Chevalier, Renaud Philippe

(56) Documents cités:
- WO-A1-2014/089198
- US-A1- 2012 078 256
- US-A1- 2014 207 142
- US-A1- 2015 190 147

## Description

La présente invention concerne un ancillaire de rétroperçage pour former un logement dans un os. De plus, la présente invention concerne un ensemble de préparation osseuse comprenant un tel ancillaire de rétroperçage.

La présente invention s'applique notamment au domaine de la chirurgie orthopédique. En particulier, la présente invention peut s'appliquer au rétroperçage d'un logement pour loger un greffon lors d'une opération de reconstruction ou de remplacement ligamentaire, par exemple pour le genou.

Le document WO 2014/089198 A1 décrit un ancillaire de rétroperçage comprenant i) un outil de coupe ayant une partie de coupe et une tige solidaire en translation de la partie de coupe, et ii) un alésoir ayant un canal pour guider la tige en translation. L'outil de coupe de WO2014089198A1 comprend i) des lames déplaçables, et ii) un mécanisme de déplacement pour déplacer les lames entre une configuration de rangement dans le canal et une configuration de coupe.

Cependant, l'ancillaire de rétroperçage de WO 2014/089198 A1 est relativement complexe à manipuler et coûteux à fabriquer à cause de ses nombreux composants. En outre, il existe un risque de défaillance des lames déplaçables ou du mécanisme de déplacement, ce qui empêcherait de former le logement dans l'os par rétroperçage.

L'état de la technique peut également être illustré par l'enseignement du document US 2015/190147 qui divulgue un ancillaire de rétroperçage comprenant un alésoir pourvu à son extrémité distale d'une fenêtre latérale à travers laquelle vient se fixer latéralement un outil de coupe. Un tel ancillaire est cependant peu fiable et peu pratique du fait de cette fixation sur le côté, en latéral, de l'outil de coupe.

La présente invention a notamment pour but de résoudre, en tout ou partie, les problèmes mentionnés ci-avant.

Dans ce but, la présente invention a pour objet un ancillaire de rétroperçage, pour former un logement dans un os, l'ancillaire de rétroperçage comprenant au moins :
- un outil de coupe comprenant : i) une partie de coupe configurée pour couper de l'os de façon à former le logement, et ii) une tige solidaire en translation de la partie de coupe, et
- un alésoir ayant un canal configuré pour loger au moins partiellement et pour guider la tige en translation suivant une direction longitudinale, ledit alésoir présente deux extrémités opposées comprenant une extrémité proximale et une extrémité distale conformée pour forer l'os ;
l'ancillaire de rétroperçage étant remarquable :
en ce que l'alésoir a une rainure longitudinale, la rainure longitudinale débouchant sur le canal, la rainure longitudinale débouchant à l'extrémité proximale de l'alésoir, l'outil de coupe étant introduit dans l'alésoir (4) par ladite extrémité proximale
en ce que la partie de coupe est fixe par rapport à la tige, et
en ce que la partie de coupe est configurée pour dépasser hors de la rainure longitudinale lorsque la tige est guidée dans le canal suivant la direction longitudinale.

Ainsi, un tel ancillaire de rétroperçage est fiable, simple à manipuler et peu coûteux, car il comprend peu de composants.

Le logement formé par un tel ancillaire de rétroperçage a la forme d'un chambrage.

L'alésoir a une forme rectiligne et allongée selon la direction longitudinale. La tige a une forme allongée et rectiligne. L'alésoir a pour fonction de réaliser un alésage, par exemple un perçage. Comme l'alésoir a rainure longitudinale, l'alésoir forme un alésoir rainuré.

Afin que la partie de coupe dépasse hors de la rainure longitudinale, les hauteurs cumulées de la tige et de la partie de coupe peuvent être supérieures à la profondeur de la rainure longitudinale.

La partie de coupe étant fixe par rapport à la tige, la partie de coupe n'a pas besoin d'être déployée, car elle dépasse hors de la rainure, ce qui rend l'ancillaire de rétroperçage particulièrement fiable.

Selon une variante, la partie de coupe est solidaire de l'extrémité distale de la tige.

Selon une variante, la partie de coupe est monobloc avec la tige.

Selon un mode de réalisation, la partie de coupe s'étend globalement suivant une direction de coupe, la direction de coupe formant avec la tige un angle compris entre 80 degrés et 100 degrés, par exemple égal à 90 degrés.

En d'autres termes, l'outil de coupe a sensiblement la forme d'un « L ».

Selon un mode de réalisation, l'alésoir comprend une région d'extrémité distale qui est configurée pour forer l'os, la région d'extrémité distale ayant une longueur comprise entre 180 mm et 220 mm, mesurée parallèlement à la direction longitudinale.

Ainsi, une telle région d'extrémité distale peut dégager un passage pour l'alésoir. Le chirurgien peut former un logement ayant une profondeur adaptée pour fixer un greffon. En effet, le chirurgien peut creuser l'os avec la partie de coupe jusqu'à ce que la région d'extrémité distale affleure sensiblement la surface externe de l'os. Alors, la profondeur du logement est égale à la longueur de la région d'extrémité distale plus la longueur de la partie de coupe, mesurées suivant la direction longitudinale.

Selon un mode de réalisation, la région d'extrémité distale a une portion de butée agencée pour arrêter l'extrémité distale de l'outil de coupe en translation dans le canal et suivant la direction longitudinale.

Selon un mode de réalisation, la portion de butée obture la rainure longitudinale qui ne débouche pas sur l'extrémité distale de l'alésoir.

Avantageusement, la longueur de l'outil de coupe est dimensionnée de sorte que l'outil de coupe dépasse du côté proximal du canal lorsque l'extrémité distale de l'outil de coupe bute contre la portion de butée.

Selon un mode de réalisation, l'alésoir a une partie distale qui a une surface externe globalement cylindrique à base circulaire et de diamètre inférieur à 6 mm, la partie distale s'étendant sur au moins 50% de la longueur de l'alésoir.

Ainsi, un tel diamètre externe relativement petit permet de couper le moins possible de tissu osseux.

Selon un mode de réalisation, l'extrémité proximale de l'alésoir a une surface proximale de guidage qui est évasée de façon à guider une extrémité distale de l'outil de coupe dans le canal et dans la rainure longitudinale.

Selon une variante, la surface proximale de guidage est au moins partiellement tronconique.

Selon un mode de réalisation, la surface externe de l'alésoir a une section d'accrochage qui est configurée pour solidariser en rotation un actionneur rotatif à l'alésoir.

Selon un mode de réalisation, la section d'accrochage comprend au moins un méplat, de préférence trois méplats répartis autour du canal.

Selon une variante, la section transversale de la section d'accrochage est plus grande que la section transversale de la partie distale.

Selon une variante, les méplats sont répartis de sorte que les centres de deux méplats consécutifs sont séparés d'un angle environ égal à 120 degrés autour du canal, donc suivant la périphérie de la surface d'accrochage.

Selon un mode de réalisation, la partie de coupe a une portion proximale ayant globalement un profil triangulaire, et la partie de coupe a une portion distale ayant globalement un profil carré.

Selon une variante, le profil triangulaire est composé d'une facette d'attaque et d'une facette de dépouille, la facette d'attaque et la facette de dépouille s'étendant de part et d'autre d'un plan médian, un angle d'attaque formé entre la facette d'attaque et le plan médian étant supérieur à un angle de dépouille formé entre la facette de dépouille et le plan médian.

Selon une variante, l'angle d'attaque est compris entre 30 degrés et 90 degrés. L'angle de dépouille est compris entre 45 degrés et 75 degrés. L'angle d'attaque et l'angle de dépouille forment l'angle de coupe pour le profil triangulaire de la partie de coupe.

Selon un mode de réalisation, l'ancillaire de rétroperçage comprend une pluralité d'outils de coupe, par exemple dix outils de coupe,
chaque outil de coupe comprenant : i) une partie de coupe configurée pour couper de l'os de façon à former le logement, et ii) une tige solidaire en translation de la partie de coupe, la partie de coupe étant configurée pour dépasser hors de la rainure longitudinale lorsque la tige est guidée dans le canal suivant la direction longitudinale,
les outils de coupe étant dimensionnés de sorte que les parties de coupe ont des hauteurs différentes.

Selon une variante, la broche de guidage est rectiligne. Avantageusement, la longueur de la broche de guidage est plus grande que la longueur du canal. Ainsi, le chirurgien peut facilement manipuler la broche de guidage, notamment pour retirer la broche de guidage de l'alésoir.

Par ailleurs, la présente invention a pour objet un ensemble de préparation osseuse, comprenant un ancillaire de rétroperçage selon l'invention, l'ensemble de préparation osseuse comprenant en outre une broche de guidage configurée pour être insérée dans l'os, la broche de guidage étant plus longue que l'alésoir,
le canal débouchant à une extrémité distale de l'alésoir de sorte que la broche de guidage peut d'abord guider l'alésoir à travers l'os puis être guidée à travers le canal jusqu'à être retirée par l'extrémité proximale de l'alésoir.

Les modes de réalisation et les variantes mentionnés ci-avant peuvent être pris isolément ou selon toute combinaison techniquement possible.

La présente invention sera bien comprise et ses avantages ressortiront aussi à la lumière de la description qui va suivre, donnée uniquement à titre d'exemple non limitatif et faite en référence aux figures schématiques annexées, dans lesquelles des signes de références identiques correspondent à des éléments structurellement et/ou fonctionnellement identiques ou similaires. Dans les figures schématiques annexées :
- la figure 1 est une vue en perspective d'une partie d'un ancillaire de rétroperçage conforme à l'invention, dans une configuration intermédiaire ;
- la figure 2 est une vue en perspective, suivant un angle différent de la figure 1, de la partie de l'ancillaire de rétroperçage de la figure 1, dans une configuration de coupe ;
- la figure 3 est une vue du détail III à la figure 2 ;
- la figure 4 est une vue similaire à la figure 2, avec un angle de perspective différent ;
- la figure 5 est une vue en perspective d'une partie de l'ancillaire de rétroperçage de la figure 1 ;
- la figure 6 est une vue en perspective d'un alésoir appartenant à l'ancillaire de rétroperçage de la figure 1 ;
- la figure 7 est une vue en perspective de l'alésoir de la figure 6, suivant un angle sensiblement opposé à l'angle de la figure 6 ;
- la figure 8 est une vue en perspective de l'alésoir de la figure 6, suivant un angle différent de la figure 6 ;
- la figure 9 est une vue d'un outil de coupe appartenant à l'ancillaire de rétroperçage de la figure 1 ;
- la figure 10 est une vue en perspective d'une partie de coupe de l'outil de coupe de la figure 9 ;
- la figure 11 est une vue en perspective de la partie de coupe de la figure 9, suivant un angle différent de la figure 10 ;
- la figure 12 est une vue de face d'une pluralité d'outils de coupe pour équiper l'ancillaire de rétroperçage de la figure 1 ;
- les figures 13 à 18 illustrent un procédé de préparation osseuse mis en oeuvre à l'aide d'un ensemble de préparation osseuse conforme à l'invention ; en particulier, la figure 13 est une vue en perspective des os d'un genou, lors d'une première étape d'un procédé de rétroperçage ;
- la figure 14 est une vue similaire à la figure 13, lors d'une étape ultérieure du procédé de rétroperçage ;
- la figure 15 est une vue similaire à la figure 14, lors d'une étape ultérieure du procédé de rétroperçage ;
- la figure 16 est une vue similaire à la figure 15, lors d'une étape ultérieure du procédé de rétroperçage ;
- la figure 17 est une vue similaire à la figure 16, lors d'une étape ultérieure du procédé de rétroperçage ; et
- la figure 18 est une vue similaire à la figure 17, lors d'une étape ultérieure du procédé de rétroperçage.

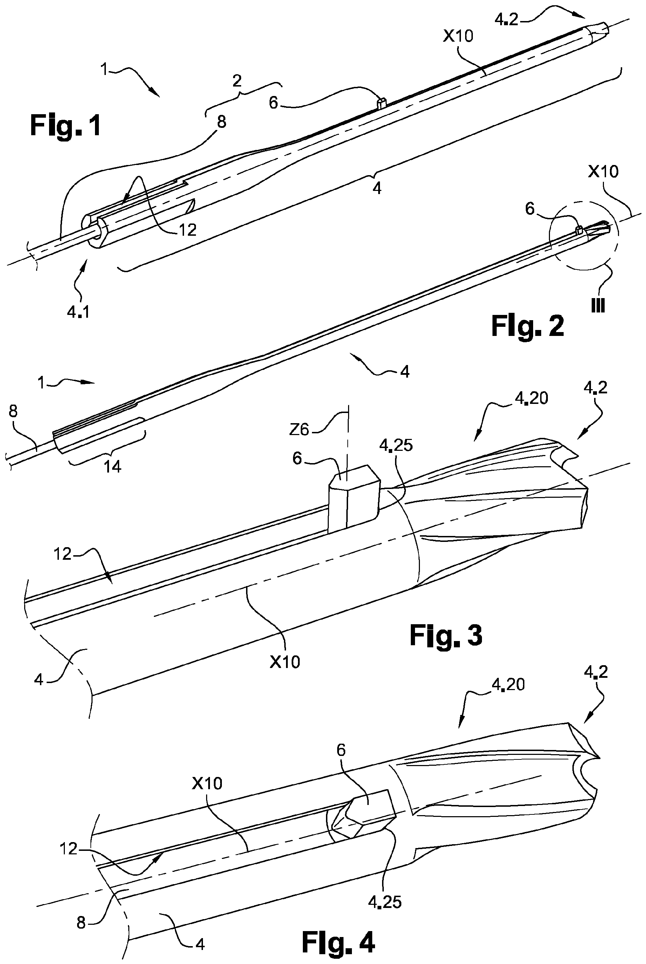

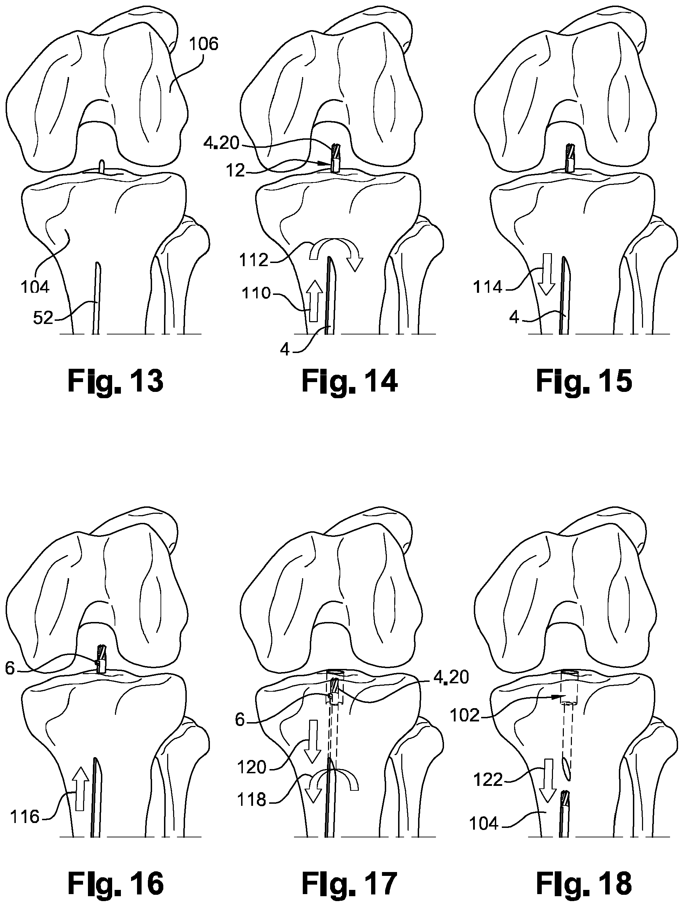

Les figures 1, 2, 3, 4 et 5 illustrent un ancillaire de rétroperçage 1 qui comprend un outil de coupe 2 et un alésoir 4. L'ancillaire de rétroperçage 1 est destiné à former un logement 102, visible à la figure 18, dans un os, ici dans un tibia 104. Le logement 102 a la forme d'un chambrage.

L'outil de coupe 2 comprend une partie de coupe 6 configurée pour couper de l'os de façon à former le logement 102. De plus, l'outil de coupe 2 comprend une tige 8 qui est solidaire en translation de la partie de coupe 6. La tige 8 a une forme rectiligne et globalement allongée suivant une ligne longitudinale X8.

La partie de coupe 6 est fixe par rapport à la tige 8. De plus, la partie de coupe 6 est solidaire de l'extrémité distale 8.2 de la tige 8. Dans l'exemple des figures 1 à 5, la partie de coupe 6 est monobloc avec la tige 8.

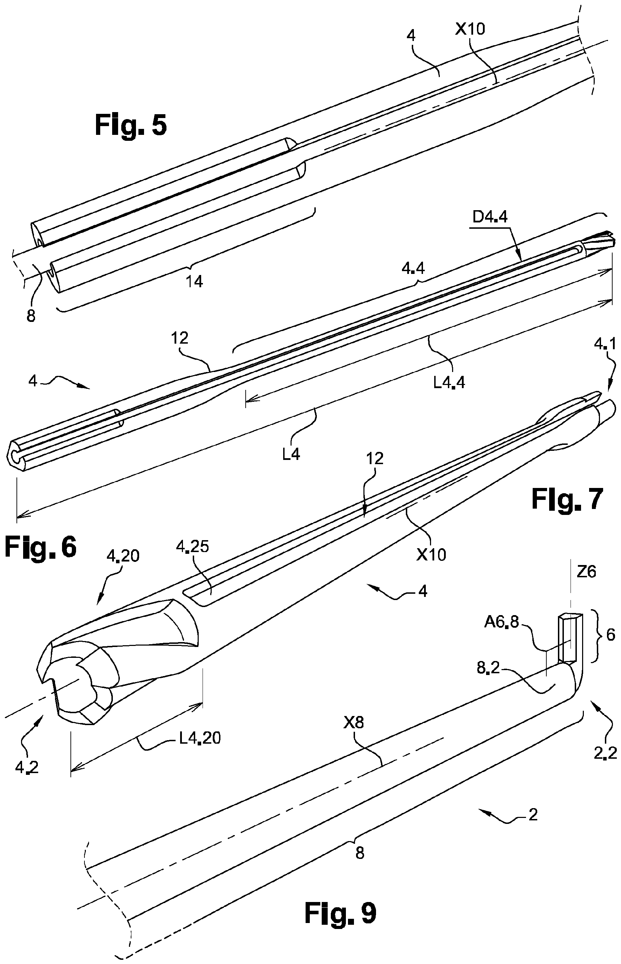

Comme le montre la figure 9, la partie de coupe 6 s'étend globalement suivant une direction de coupe Z6. La direction de coupe Z6 forme avec la tige 8 un angle A6.8 qui est ici environ égal à 90 degrés. L'outil de coupe 2 a donc sensiblement la forme d'un « L ».

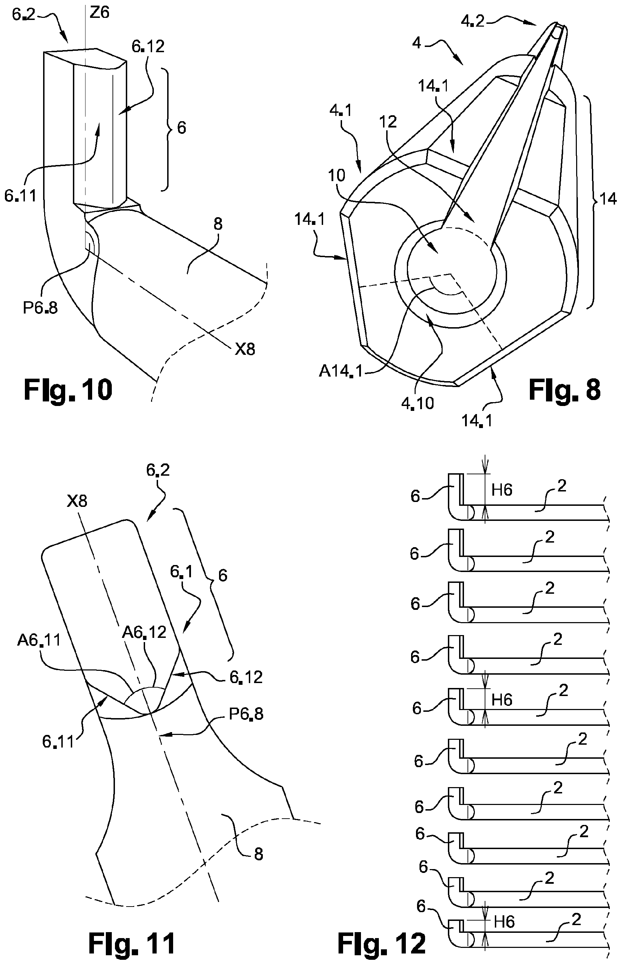

Comme le montrent les figures 10 et 11, la partie de coupe 6 a une portion proximale 6.1 et une portion distale 6.2. La portion proximale 6.1 a globalement un profil triangulaire. La portion distale 6.2 a globalement un profil carré. La portion distale 6.2 est configurée pour couper de l'os afin de permettre la pénétration de l'outil de coupe 2 dans le tibia 104 lors du guidage de la tige 8 dans le canal 10.

Le profil triangulaire est composé d'une facette d'attaque 6.11 et d'une facette de dépouille 6.12. La facette d'attaque 6.11 et la facette de dépouille 6.12 s'étendent de part et d'autre d'un plan médian P6.8 qui contient la ligne longitudinale X8 et la direction de coupe Z6.

Un angle d'attaque A6.11 formé entre la facette d'attaque 6.11 et le plan médian P6.8 peut être supérieur à un angle de dépouille A6.12 formé entre la facette de dépouille 6.12 et le plan médian P6.8. En l'occurrence, l'angle d'attaque A6.11 est environ égal à 55 degrés et l'angle de dépouille A6.12 est environ égal à 45 degrés. L'angle d'attaque A6.11 et l'angle de dépouille A6.12 forment ensemble l'angle de coupe pour le profil triangulaire de la partie de coupe.

L'alésoir 4 présente deux extrémités opposées, à savoir une extrémité proximale 4.1 et une extrémité distale 4.2 conformée pour forer l'os.

L'alésoir 4 a un canal 10 qui est visible sur les figures 6, 7 et 8. Le canal 10 est configuré pour loger au moins partiellement la tige 8 en translation suivant une direction longitudinale X10. Le canal 10 est configuré pour guider la tige 8 en translation suivant la direction longitudinale X10. L'alésoir 4 a une forme rectiligne et allongée selon la direction longitudinale X10.

Comme le montrent les figures 6 et 7, l'alésoir 4 a une rainure longitudinale 12. La rainure longitudinale 12 débouche sur le canal 10. La rainure longitudinale 12 débouche à l'extrémité proximale 4.1 de l'alésoir 4.

La partie de coupe 6 est configurée pour dépasser hors de la rainure longitudinale 12 lorsque la tige 8 est guidée dans le canal 10 suivant la direction longitudinale X10. À cet effet, les hauteurs cumulées de la tige 8 et de la partie de coupe 6, mesurées selon la direction de coupe Z6, peuvent être supérieures à la profondeur de la rainure longitudinale 12.

L'alésoir 4 comprend une région d'extrémité distale 4.20 qui est configurée pour forer l'os. Dans l'exemple des figures 1 à 8, l'alésoir 4 a la forme d'une mèche de foret de perçage. La région d'extrémité distale 4.20 a ici une longueur L4.20 environ égale à 200 mm, mesurée parallèlement à la direction longitudinale X10 et à partir de l'extrémité distale 4.2.

La région d'extrémité distale 4.20 a une portion de butée 4.25 agencée pour arrêter l'extrémité distale 2.2 de l'outil de coupe 2 en translation dans le canal 10 et suivant la direction longitudinale X10. En d'autres termes, la portion de butée 4.25 obture la rainure longitudinale 12, c'est-à-dire que cette portion de butée 4.25 constitue le fond de la rainure longitudinale 12 qui ne débouche pas sur l'extrémité distale 4.2 de l'alésoir 4 tandis qu'elle débouche pour rappel sur l'extrémité proximale 4.1 de l'alésoir 4.

Le canal 10 débouche à une extrémité distale 4.2 de l'alésoir 4. Donc le canal 10 débouche hors de la région d'extrémité distale 4.20, de sorte qu'une broche de guidage 52, visible à la figure 13, peut d'abord guider l'alésoir 4 à travers l'os 104 puis de sorte que la broche de guidage 52 peut être guidée à travers le canal 10 jusqu'à être retirée par l'extrémité proximale 4.1 de l'alésoir 4.

Par ailleurs, l'alésoir 4 a une partie distale 4.4 qui a une surface externe globalement cylindrique à base circulaire et de diamètre D4.4 environ égal à 4,5 mm. La partie distale 4.4 s'étend ici sur environ 60% de la longueur totale L4 de l'alésoir 4. En d'autres termes, la longueur L4.4 de la partie distale 4.4 représente environ 60% de la longueur totale L4 de l'alésoir 4, comme le montre la figure 6.

Comme le montre la figure 8, l'extrémité proximale 4.1 de l'alésoir 4 a une surface proximale de guidage 4.10. La surface proximale de guidage 4.10 est évasée pour guider une extrémité distale 2.2 de l'outil de coupe 2 dans le canal 10 et dans la rainure longitudinale 12. La surface proximale de guidage 4.10 est ici tronconique et s'étend sur un secteur angulaire environ égal à 300 degrés. La surface proximale de guidage 4.10 peut être une surface de révolution (360 degrés) formée par usinage, puis partiellement coupée lors de l'usinage de la rainure longitudinale 12.

La surface externe de l'alésoir 4 a une section d'accrochage 14 qui est configurée pour solidariser en rotation un actionneur rotatif non représenté à l'alésoir 4. Par ailleurs, la section d'accrochage 14.1 a une plus grande superficie transversale que la partie distale 4.4 de l'alésoir 4. La section d'accrochage 14 est ici non circulaire.

La section d'accrochage 14 comprend trois méplats 14.1 qui sont répartis autour du canal 10. Les trois méplats 14.1 sont répartis de sorte que les centres de deux méplats 14.1 consécutifs sont séparés d'un angle A14.1 environ égal à 120 degrés autour du canal 10, donc suivant la périphérie de la surface d'accrochage 14.

La figure 12 illustre une pluralité d'outils de coupe 2 destinés à équiper l'ancillaire de rétroperçage 1. La pluralité d'outils de coupe 2 comprend ici dix outils de coupe 2. Les outils de coupe 2 sont dimensionnés de sorte que les parties de coupe 6 des outils de coupe ont des hauteurs H6 différentes. En d'autres termes, les parties de coupe 6 ont des hauteurs différentes deux à deux. Les hauteurs H6 sont mesurées parallèlement à la direction de coupe Z6. La pluralité d'outils de coupe 2 permet de forer un logement adapté aux dimensions du greffon prélevé.

Les figures 13, 14, 15, 16, 17 et 18 illustrent un ensemble de préparation osseuse comprenant l'ancillaire de rétroperçage 1 et une broche de guidage 52 qui est rectiligne. La broche de guidage 52 est configurée pour être insérée dans le tibia 104.

La broche de guidage 52 est plus longue que l'alésoir 4. La section transversale de la broche de guidage 52 est plus petite que la section transversale du canal 10. Ainsi, la broche de guidage 52 peut être déplacée en translation dans le canal 10.

Lorsque l'ensemble de préparation osseuse est en service, un chirurgien peut suivre un procédé de préparation osseuse comprenant les étapes décrites ci-après.

Au cours d'une première étape (figure 13), le chirurgien insère la broche de guidage 52 à travers le tibia 104 jusqu'à faire déboucher la broche de guidage 52 entre le tibia 104 et le fémur 106, comme le montre la figure 1.

Au cours d'une deuxième étape (figure 14), le chirurgien place l'extrémité proximale de la broche de guidage 52 dans le canal 10. Puis le chirurgien guide l'alésoir 4 autour de la broche de guidage 52 et à travers le tibia 104, suivant la flèche 110. Simultanément, le chirurgien fore le tibia 104 au moyen de la région d'extrémité distale 4.20, suivant la flèche 112.

Au cours d'une troisième étape (figure 15), le chirurgien retire la broche de guidage 52 du canal 10, donc de l'alésoir 4, en tirant la broche de guidage 52 par l'extrémité proximale 4.1, suivant la flèche 114. Après cette étape, le canal 10 est vide.

Au cours d'une quatrième étape (figure 16), le chirurgien introduit l'outil de coupe 2 dans l'alésoir 4 par l'extrémité proximale 4.1, suivant la flèche 116. Lors de cette étape, la partie de coupe 6 s'engage dans la rainure longitudinale 12 qui débouche à l'extrémité proximale 4.1 de l'alésoir 4 pour permettre justement d'insérer cette partie de coupe 6 qui sera ensuite guidée dans cette rainure longitudinale 12. En outre, lors de cette étape, la tige 8 est guidée en translation dans le canal 10, et la partie de coupe 6 découpe son propre passage dans le tibia 104 tout en avançant dans la rainure longitudinale 12 en direction de l'extrémité distale 4.2 de l'alésoir 4.

Au cours d'une cinquième étape non représentée, le chirurgien accroche un actionneur rotatif sur la surface d'accrochage 14, de façon à pouvoir entraîner en rotation l'ancillaire de rétroperçage 1.

Au cours d'une sixième étape (figure 16), le chirurgien entraîne en rotation l'ancillaire de rétroperçage 1 autour de la direction longitudinale X10, suivant la flèche 118, en ramenant l'ancillaire de rétroperçage 1 avec la partie de coupe 6 dans le tibia 104, suivant la flèche 120. Au cours de cette étape, la partie de coupe 6 usine le logement 102 dans le tibia 104.

Au cours d'une septième étape non représentée, le chirurgien aligne la partie de coupe avec le passage que la partie de coupe a découpé dans le tibia 104.

Au cours d'une huitième étape (figure 18), le chirurgien retire l'ancillaire de rétroperçage 1 du tibia 104, en translation suivant la direction longitudinale X10 et la flèche 122.

Bien entendu, la présente invention n'est pas limitée aux modes de réalisation particuliers décrits dans la présente demande de brevet, ni à des modes de réalisation à la portée de l'homme du métier. D'autres modes de réalisation peuvent être envisagés sans sortir du cadre de l'invention, à partir de tout élément équivalent à un élément indiqué dans la présente demande de brevet.

## Revendications

1. Ancillaire de rétroperçage (1), pour former un logement (102) dans un os, l'ancillaire de rétroperçage (1) comprenant au moins :
- un outil de coupe (2) comprenant : i) une partie de coupe (6) configurée pour couper de l'os de façon à former le logement (102), et ii) une tige (8) solidaire en translation de la partie de coupe (6), et
- un alésoir (4) ayant un canal (10) configuré pour loger au moins partiellement et pour guider la tige (8) en translation suivant une direction longitudinale (X10), ledit alésoir (4) présente deux extrémités opposées comprenant une extrémité proximale (4.1) et une extrémité distale (4.2) conformée pour forer l'os, l'alésoir (4) payant une rainure longitudinale (12), la rainure longitudinale (12) débouchant sur le canal (10), la rainure longitudinale (12) débouchant à l'extrémité proximale de l'alésoir (4), l'outil de coupe (2) étant introduit dans l'alésoir (4) par ladite extrémité proximale,
et
la partie de coupe (6) étant configurée pour dépasser hors de la rainure longitudinale (12) lorsque la tige (8) est guidée dans le canal (10) suivant la direction longitudinale (X10); l'ancillaire de rétroperçage (1) étant **caractérisé : en ce que** la partie de coupe (6) est fixe par rapport à la tige (8).

2. Ancillaire de rétroperçage (1) selon la revendication 1, dans lequel la partie de coupe (6) s'étend globalement suivant une direction de coupe (Z6), la direction de coupe (Z6) formant avec la tige (8) un angle (A6.8) compris entre 80 degrés et 100 degrés, par exemple égal à 90 degrés.

3. Ancillaire de rétroperçage (1) selon l'une quelconque des revendications précédentes, dans lequel l'alésoir (4) comprend une région d'extrémité distale (4.20) qui est configurée pour forer l'os, la région d'extrémité distale (4.20) ayant une longueur (L4.20) comprise entre 180 mm et 220 mm, mesurée parallèlement à la direction longitudinale (X10) et à partir de l'extrémité distale (4.2).

4. Ancillaire de rétroperçage (1) selon la revendication précédente, dans lequel la région d'extrémité distale (4.20) a une portion de butée (4.25) agencée pour arrêter l'extrémité distale (2.2) de l'outil de coupe (2) en translation dans le canal (10) et suivant la direction longitudinale (X10).

5. Ancillaire de rétroperçage (1) selon la revendication 4, dans lequel la portion de butée (4.25) obture la rainure longitudinale (12) qui ne débouche pas sur l'extrémité distale (4.2) de l'alésoir (4).

6. Ancillaire de rétroperçage (1) selon l'une quelconque des revendications précédentes, dans lequel l'alésoir (4) a une partie distale (4.4) qui a une surface externe globalement cylindrique à base circulaire et de diamètre (D4.4) inférieur à 6 mm, la partie distale (4.4) s'étendant sur au moins 50% de la longueur (L4) de l'alésoir (4).

7. Ancillaire de rétroperçage (1) selon l'une quelconque des revendications précédentes, dans lequel l'extrémité proximale (4.1) de l'alésoir (4) a une surface proximale de guidage (4.10) qui est évasée de façon à guider une extrémité distale (2.2) de l'outil de coupe (2) dans le canal (10) et dans la rainure longitudinale (12).

8. Ancillaire de rétroperçage (1) selon l'une quelconque des revendications précédentes, dans lequel la surface externe de l'alésoir (4) a une section d'accrochage (14) qui est configurée pour solidariser en rotation un actionneur rotatif à l'alésoir (4).

9. Ancillaire de rétroperçage (1) selon la revendication précédente, dans lequel la section d'accrochage (14) comprend au moins un méplat (14.1), de préférence trois méplats (14.1) répartis autour du canal (10).

10. Ancillaire de rétroperçage (1) selon l'une quelconque des revendications précédentes, dans lequel la partie de coupe (6) a une portion proximale (6.1) ayant globalement un profil triangulaire, et dans lequel la partie de coupe (6) a une portion distale (6.2) ayant globalement un profil carré.

11. Ancillaire de rétroperçage (1) selon l'une quelconque des revendications précédentes, comprenant une pluralité d'outils de coupe (2), par exemple dix outils de coupe (2),
chaque outil de coupe (2) comprenant : i) une partie de coupe (6) configurée pour couper de l'os de façon à former le logement (102), et ii) une tige (8) solidaire en translation de la partie de coupe (6), la partie de coupe (6) étant configurée pour dépasser hors de la rainure longitudinale (12) lorsque la tige (8) est guidée dans le canal (10) suivant la direction longitudinale (X10),
les outils de coupe (2) étant dimensionnés de sorte que les parties de coupe (6) ont des hauteurs (H6) différentes.

12. Ensemble de préparation osseuse, comprenant un ancillaire de rétroperçage (1) selon l'une quelconque des revendications précédentes, l'ensemble de préparation osseuse comprenant en outre une broche de guidage (52) configurée pour être insérée dans l'os, la broche de guidage (52) étant plus longue que l'alésoir (4),
le canal (10) débouchant à une extrémité distale (4.2) de l'alésoir (4) de sorte que la broche de guidage (52) peut d'abord guider l'alésoir (4) à travers l'os puis être guidée à travers le canal (10) jusqu'à être retirée par l'extrémité proximale (4.1) de l'alésoir (4).

## Patentansprüche

1. Hilfsmittel zum retrograden Anbohren (1), um eine Aufnahme (102) in einem Knochen zu bilden, wobei das Hilfsmittel zum retrograden Anbohren (1) mindestens umfasst:
- ein Schneidwerkzeug (2), umfassend: i) einen Schneidbereich (6), der dafür ausgestaltet ist, Knochen so zu schneiden, dass die Aufnahme (102) gebildet wird, und ii) einen Schaft (8), der translatorisch fest mit dem Schneidbereich (6) verbunden ist, und
- eine Reibahle (4), die einen Kanal (10) aufweist, welcher dafür ausgestaltet ist, den Schaft (8) mindestens teilweise aufzunehmen und translatorisch entlang einer Längsrichtung (X10) zu führen, wobei die Reibahle (4) zwei gegenüberliegende Enden aufweist, die ein proximales Ende (4.1) und ein distales Ende (4.2) umfassen, die dafür ausgebildet sind, den Knochen zu bohren,
wobei die Reibahle (4) eine Längsnut (12) aufweist, wobei die Längsnut (12) in den Kanal (10) mündet, wobei die Längsnut (12) am proximalen Ende der Reibahle (4) mündet, wobei das Schneidwerkzeug (2) über das proximale Ende in die Reibahle (4) eingeführt wird, und wobei der Schneidbereich (6) dafür ausgestaltet ist, aus der Längsnut (12) herauszutreten, wenn der Schaft (8) im Kanal (10) entlang der Längsrichtung (X10) geführt wird; wobei das Hilfsinstrument zum retrograden Anbohren (1) **gekennzeichnet ist: dadurch**, dass der Schneidbereich (6) in Bezug auf den Schaft (8) fest ist.

2. Hilfsinstrument zum retrograden Anbohren (1) nach Anspruch 1, wobei sich der Schneidbereich (6) allgemein entlang einer Schneidrichtung (Z6) erstreckt, wobei die Schneidrichtung (Z6) einen Winkel (A6.8) im Bereich zwischen 80 Grad und 100 Grad, zum Beispiel von gleich 90 Grad, zum Schaft (8) bildet.

3. Hilfsinstrument zum retrograden Anbohren (1) nach einem der vorstehenden Ansprüche, wobei die Reibahle (4) eine distale Endregion (4.20) umfasst, die dafür ausgestaltet ist, den Knochen zu bohren, wobei die distale Endregion (4.20) parallel zur Längsrichtung (X10) und vom distalen Ende (4.2) ausgehend gemessen eine Länge (L4.20) im Bereich zwischen 180 mm und 220 mm aufweist.

4. Hilfsinstrument zum retrograden Anbohren (1) nach dem vorstehenden Anspruch, wobei die distale Endregion (4.20) einen Anschlagabschnitt (4.25) aufweist, der dafür eingerichtet ist, das distale Ende (2.2) des Schneidwerkzeugs (2) translatorisch im Kanal (10) und entlang der Längsrichtung (X10) anzuhalten.

5. Hilfsinstrument zum retrograden Anbohren (1) nach Anspruch 4, wobei der Anschlagabschnitt (4.25) die Längsnut (12), die nicht in das distale Ende (4.2) der Reibahle (4) mündet, verschließt.

6. Hilfsinstrument zum retrograden Anbohren (1) nach einem der vorstehenden Ansprüche, wobei die Reibahle (4) einen distalen Bereich (4.4) aufweist, der eine allgemein zylindrische Außenfläche mit kreisförmiger Basis und eines Durchmessers (D4.4) von kleiner als 6 mm aufweist, wobei sich der distale Bereich (4.4) auf mindestens 50 % der Länge (L4) der Reibahle (4) erstreckt.

7. Hilfsinstrument zum retrograden Anbohren (1) nach einem der vorstehenden Ansprüche, wobei das proximale Ende (4.1) der Reibahle (4) eine proximale Führungsfläche (4.10) aufweist, die aufgeweitet ist, sodass ein distales Ende (2.2) des Schneidwerkzeugs (2) im Kanal (10) und in der Längsnut (12) geführt wird.

8. Hilfsinstrument zum retrograden Anbohren (1) nach einem der vorstehenden Ansprüche, wobei die Außenfläche der Reibahle (4) eine Verankerungssektion (14) aufweist, die dafür ausgestaltet ist, einen Drehantrieb drehfest mit der Reibahle (4) zu verbinden.

9. Hilfsinstrument zum retrograden Anbohren (1) nach dem vorstehenden Anspruch, wobei die Verankerungssektion (14) mindestens eine Abflachung (14.1), vorzugsweise drei Abflachungen (14.1) umfasst, die um den Kanal (10) herum verteilt sind.

10. Hilfsinstrument zum retrograden Anbohren (1) nach einem der vorstehenden Ansprüche, wobei der Schneidbereich (6) einen proximalen Abschnitt (6.1) aufweist, welcher allgemein ein dreieckiges Profil aufweist, und wobei der Schneidbereich (6) einen distalen Abschnitt (6.2) aufweist, welcher allgemein ein viereckiges Profil aufweist.

11. Hilfsinstrument zum retrograden Anbohren (1) nach einem der vorstehenden Ansprüche, umfassend eine Vielzahl von Schneidwerkzeugen (2), zum Beispiel zehn Schneidwerkzeuge (2),
wobei jedes Schneidwerkzeug (2) umfasst: i) einen Schneidbereich (6), der dafür ausgestaltet ist, Knochen so zu schneiden, dass die Aufnahme (102) gebildet wird, und ii) einen Schaft (8), der translatorisch fest mit dem Schneidbereich (6) verbunden ist, wobei der Schneidbereich (6) dafür ausgestaltet ist, aus der Längsnut (12) herauszutreten, wenn der Schaft (8) im Kanal (10) entlang der Längsrichtung (X10) geführt wird,
wobei die Schneidwerkzeuge (2) derart bemessen sind, dass die Schneidbereiche (6) verschiedene Höhen (H6) aufweisen.

12. Knochenvorbereitungssatz, der ein Hilfsmittel zum retrograden Anbohren (1) nach einem der vorstehenden Ansprüche umfasst, wobei der Knochenvorbereitungssatz weiter einen Führungsstift (52) umfasst, der dafür ausgestaltet ist, in den Knochen eingefügt zu werden, wobei der Führungsstift (52) länger ist als die Reibahle (4),
wobei der Kanal (10) an einem distalen Ende (4.2) der Reibahle (4) mündet, derart, dass der Führungsstift (52) zunächst die Reibahle (4) durch den Knochen hindurch führen kann, und danach durch den Kanal (10) hindurch geführt werden kann, bis er vom proximalen Ende (4.1) der Reibahle (4) zurückgezogen wird.

## Claims

1. A back-drilling ancillary (1), for forming a recess (102) in a bone, the back-drilling ancillary (1) comprising at least:
- one cutting tool (2) comprising: i) a cutting portion (6) configured to cut bone so as to form the recess (102), and ii) a rod (8) secured in translation to the cutting portion (6), and
- one reamer (4) having a channel (10) configured to house, at least partially, and to guide the rod (8) in translation in a longitudinal direction (X10), said reamer (4) has two opposite ends comprising a proximal end (4.1) and a distal end (4.2) shaped so as to drill the bone,
the reamer (4) having a longitudinal groove (12), the longitudinal groove (12) opening onto the channel (10), the longitudinal groove (12) opening at the proximal end of the reamer (4), the cutting tool (2) being introduced into the reamer (4) by said proximal end, and
the cutting portion (6) being configured to project out of the longitudinal groove (12) when the rod (8) is guided in the channel (10) along the longitudinal direction (X10); the back-drilling ancillary (1) being **characterized in that** the cutting portion (6) is fixed relative to the rod (8).

2. The back-drilling ancillary (1) according to claim 1, wherein the cutting portion (6) generally extends along a cutting direction (Z6), the cutting direction (Z6) forming with the rod (8) an angle (A6.8) comprised between 80 degrees and 100 degrees, for example equal to 90 degrees.

3. The back-drilling ancillary (1) according to any one of the preceding claims, wherein the reamer (4) comprises a distal end region (4.20) which is configured to drill the bone, the distal end region (4.20) having a length (L4.20) comprised between 180 mm and 220 mm, measured parallel to the longitudinal direction (X10) and from the distal end (4.2).

4. The back-drilling ancillary (1) according to the preceding claim, wherein the distal end region (4.20) has a stop portion (4.25) arranged to stop the distal end (2.2) of the cutting tool (2) in translation in the channel (10) and along the longitudinal direction (X10).

5. The back-drilling ancillary (1) according to claim 4, wherein the stop portion (4.25) obturates the longitudinal groove (12) which does not open onto the distal end (4.2) of the reamer (4).

6. The back-drilling ancillary (1) according to any one of the preceding claims, wherein the reamer (4) has a distal portion (4.4) which has a generally cylindrical outer surface with a circular base and with a diameter (D4.4) smaller than 6 mm, the distal portion (4.4) extending over at least 50% of the length (L4) of the reamer (4).

7. The back-drilling ancillary (1) according to any one of the preceding claims, wherein the proximal end (4.1) of the reamer (4) has a proximal guide surface (4.10) which is flared so as to guide a distal end (2.2) of the cutting tool (2) in the channel (10) and in the longitudinal groove (12).

8. The back-drilling ancillary (1) according to any one of the preceding claims, wherein the outer surface of the reamer (4) has a hooking section (14) which is configured to secure in rotation a rotary actuator to the reamer (4).

9. The back-drilling ancillary (1) according to the preceding claim, wherein the hooking section (14) comprises at least one flattened portion (14.1), preferably three flattened portions (14.1) distributed around the channel (10).

10. The back-drilling ancillary (1) according to any one of the preceding claims, wherein the cutting portion (6) has a proximal portion (6.1) having a generally triangular profile, and wherein the cutting portion (6) has a distal portion (6.2) having a generally square profile.

11. The back-drilling ancillary (1) according to any one of the preceding claims, comprising a plurality of cutting tools (2), for example ten cutting tools (2),
each cutting tool (2) comprising: i) a cutting portion (6) configured to cut bone so as to form the recess (102), and ii) a rod (8) secured in translation to the cutting portion (6), the cutting portion (6) being configured to project out of the longitudinal groove (12) when the rod (8) is guided in the channel (10) along the longitudinal direction (X10),
the cutting tools (2) being sized so that the cutting portions (6) have different heights (H6).

12. A bone preparation set, comprising a back-drilling ancillary ((1) according to any one of the preceding claims, the bone preparation set further comprising a guide wire (52) configured to be inserted into the bone, the guide wire (52) being longer than the reamer (4),
the channel (10) opening at a distal end (4.2) of the reamer (4) such that the guide wire (52) can guide the reamer (4) through the bone at first and then be guided through the channel (10) until being removed by the proximal end (4.1) of the reamer (4).
